(19) Europäisches Patentamt
European Patent Office
Office européen des brevets

(11) EP 0 577 817 B2

(12) NOUVEAU FASCICULE DE BREVET EUROPEEN

(45) Date de publication et mention de la décision concernant l'opposition:
**16.04.2003 Bulletin 2003/16**

(45) Mention de la délivrance du brevet:
**17.04.1996 Bulletin 1996/16**

(21) Numéro de dépôt: **93904122.4**

(22) Date de dépôt: **27.01.1993**

(51) Int Cl.[7]: **A61K 7/48**, A61K 7/00, A61K 9/06

(86) Numéro de dépôt international:
**PCT/FR93/00078**

(87) Numéro de publication internationale:
**WO 93/014742 (05.08.1993 Gazette 1993/19)**

# (54) EMULSION EAU-DANS-HUILE STABLE AU COURS DU TEMPS A HAUTE TENEUR EN SILICONE

HOHES SILIKON-GEHALT WASSER-IN-ÖL LAGERUNGSSTABILE EMULSIONEN

WATER-IN-OIL EMULSION STABLE IN THE COURSE OF TIME, WITH HIGH SILICONE CONTENT

(84) Etats contractants désignés:
**AT BE CH DE ES FR GB GR IT LI NL PT SE**

(30) Priorité: **27.01.1992 FR 9200816**

(43) Date de publication de la demande:
**12.01.1994 Bulletin 1994/02**

(73) Titulaire: **L'OREAL**
**75008 Paris (FR)**

(72) Inventeurs:
- **BARA, Isabelle**
**F-75013 Paris (FR)**
- **MELLUL, Myriam**
**F-94240 L'Hay-les-Roses (FR)**

(74) Mandataire: **Tanty, François et al**
**Nony & Associés,**
**3, Rue de Penthièvre**
**75008 Paris (FR)**

(56) Documents cités:
**EP-A- 0 176 884**
**EP-A- 0 331 833**
**EP-A- 0 374 332**
**GB-A- 2 243 780**

- **"Cyclomethicone-containing cosmetic water-in-oil lotions based on organosilicone emulsifiers" par Peter Hameyer et Clive Gould, Tego Cosmetic - Version Imprimée d'une conférence tenue au salon INCOSMECTICS à Birmingham, 1990**
- **"Cyclomethiconhaltige kosmetische W/O-Lotionen auf Basis siliciumorganischer Emulgatoren" par Peter Hameyer, Seifen-Oele-Fette-Wachse, Edition 10/1990**
- **Abil (RTM) Silicones, TH. Goldschmidt AG (Edition 1/Janvier 1995)**
- **Harry's Cosmetology, Vol. 1: "Deodorants and Antiperspirants" pp. 262-265**

Remarques:
Le dossier contient des informations techniques présentées postérieurement au dépôt de la demande et ne figurant pas dans le présent fascicule.

EP 0 577 817 B2

## Description

La présente invention a pour objet des émulsions eau-dans-huile (E/H) à haute teneur en silicone.

De telles émulsions E/H sont utiles en cosmétique notamment pour leur aptitude à former des films à la surface de la peau prévenant efficacement la perte d'eau trans-épidermique et donnant une bonne résistance aux contaminations par les micro-organismes.

Il est connu que plus la teneur en huile de silicone augmente, plus l'obtention d'une émulsion E/H stable est difficile non seulement dans le temps mais également lorsqu'elle est soumise à d'importantes variations de température.

Malgré les recherches importantes dans ce domaine, il n'a pas été possible de mettre au point des émulsions E/H, de bonne stabilité, ayant une forte teneur en huile de silicone.

Ces problèmes de stabilité peuvent, selon le brevet US 4.698.178, être au moins partiellement résolus per l'emploi d'une nouvelle classe de tensioactifs siliconés.

Toutefois, si ce brevet indique que par l'emploi de ces tensioactifs on peut obtenir des émulsions E/H dont la pro-portion en huile de silicone peut s'élever de 8 à 50 %, celles-ci ne sont illustrées à l'exception d'un exemple que par des émulsions renfermant au maximum 8,5 % d'huile de silicone.

Selon ce brevet, la stabilité est obtenue non seulement à l'aide des tensioactifs siliconés décrits, mais également par l'usage d'une part de polyols pour les températures basses et d'autre part d'électrolytes ou de savons métalliques pour les températures élevées.

La stabilisation des émulsions E/H se réalise de manière connue à l'aide de stabilisants de la phase grasse notamment par des polymères liposolubles, des cires ou des argiles organiquement modifiées, ces dernières étant décrites dans l'EP-A-0.009.404 et l'EP-A-0.331.833.

Il a maintenant été découvert de manière inattendue et surprenante que par l'emploi de gélifiants non sensibles aux électrolytes dans la phase aqueuse dispersée en association avec un émulsionnant du type alkyl- ou alcoxy-diméthicone copolyol, il était possible de résoudre les inconvénients mentionnés ci-dessus et d'obtenir des émulsions E/H ayant une bonne stabilité dans le temps et aux variations de température.

La présente invention a donc pour objet une émulsion eau-dans-huile stable, à usage cosmétique ou pharmaceutique, caractérisée par le fait qu'elle comprend une phase grasse en une proportion de 15 à 40 % constituée d'au moins une silicone à raison de 15 à 40 % en poids par rapport au poids total de l'émulsion et une phase aqueuse contenant au moins un gélifiant aqueux organique.insensible aux électrolyles, l'agent émulsionnant de ladite émulsion étant un alkyl- ou alcoxy-diméthicone copolyol de Formule générale = choisi dans le groupe constitué par les gommes natuelles, is protéines, is hydrolysat de protéines, le dérivés amplipus, le polyéthyleinlglycols, et leurs mélanges

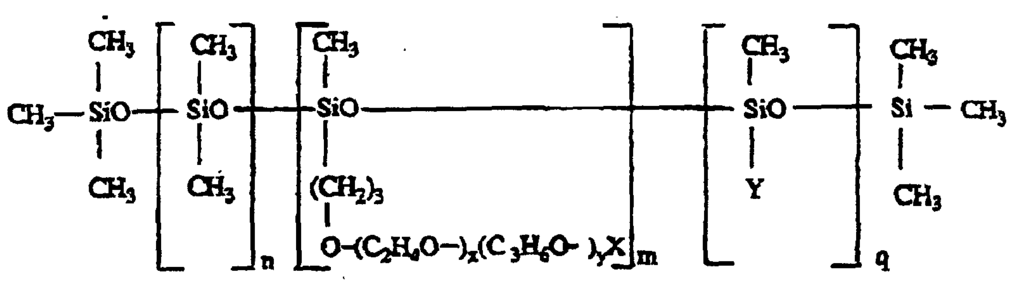

dans laquelle :

X est un atome d'hydrogène, un alkyle, un alcoxy ou un acyle, en $C_1$-$C_{16}$,
Y est un radical alkyle ou alcoxy en $C_8$ à $C_{22}$,
n = 0 à 200,
m = 1 à 40,
q = 1 à 100,

le poids moléculaire du reste $(C_2H_4O\text{-})_x(C_3H_6O\text{-})_y$-X étant de 250 à 2000, x et y étant choisis de telle sorte que le rapport en poids des groupes oxyéthylène/oxypropyiène soit compris entre 100:0 et 20:80.

L'émulsion E/H selon l'invention répond parfaitement aux normes de stabilité soit:

- résistance à l'épreuve de centrifugation à 4000 tr/mn pendant 1 heure.
- résistance au vieillissement à température ambiante pendant 3 mois ainsi qu'à 45°C et à +4°C, et
- résistance à 8 cycles successifs de 8 heures chacun dont les températures s'échelonnent de -25°C à +47°C.

L'émulsion selon l'invention répond aux critères suivants :

- elle a et conserve au cours de ces tests un aspect macroscopique et microscopique homogène et stable (globules finement dispersés, absence de relargage) et
- sa viscosité est constante au cours du temps et est comprise entre 0,1 Pa.sec et 10 Pa.sec et de préférence entre 0,2 Pa.sec et 6 Pa.sec..

Les émulsions selon l'invention possèdent par ailleurs de bonnes qualités sensorielles notamment une grande facilité d'application, du confort de la douceur, une bonne matité, de l'uniformité et de la tenue.
L'emploi des gélifiants aqueux selon l'invention confère à la phase aqueuse dispersée des propriétés Rhéologiques d'épaississement et/ou de thixotropie et permet donc d'obtenir une émulsion plus stable. Les émulsions ainsi stabilisées permettent de se passer d'agents conservateurs. En effet, de façon surprenante, on a constaté que les émulsions stabilisées présentaient d'excellentes propriétés bactériostatiques et/ou bactéricides.
Le gélifiant aqueu organique est choisi parmi :

- les gommes naturelles telles que les gommes de xanthane, de guar, de caroube, les sciéroglucanes, les dérivés de chitine ou de chitosane,
- les protéines ou leurs hydrolysats, telles que la kératine, la gélatine, le coliagène,
- les dérivés acryliques et méthacryliques, tels que le polyacrylate de glycérol (dont le produit vendu par la Société SEDERMA sous la dénomination de "LUBRAJEL ®") et le copolymère d'acrylate d'ammonium (dont le produit vendu par la Société HOECHST sous la dénomination de "PAS 5161®"),
- les polyéthylèneglycols (PEG) tels que les produits vendus par la Société UNION CARBIDE sous la dénomination de "CARBOWAX®", et
- les mélanges de ceux-ci.

Selon l'invention, le gélifiant aqueux est présent en une proportion comprise entre 0,1 et 5 % et de préférance entre 0,3 et 2 % en poids de matière active par rapport au poids total de l'émulsion.
La silicone utilisable selon l'invention peut être un polydioxganosiloxane linéaire, éventuellement fonctionnalisé, ou cyclique ou un méthylorganopolysiloxane ou un mélange de ceux-ci.
Les polydiorganosiloxanes linéaires éventuellement fonctionnalisés utilisables selon l'invention répondent à la formule générale suivante :

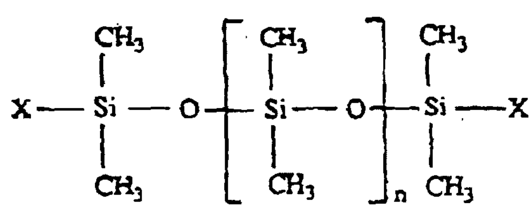

dans laquelle :

X est $-CH_3$ ou OH, et
n est 0 à 5000.

Parmi ceux-ci, on citera notamment les produits vendus sous la dénomination de "AK®" par la Société WACKER, "SF®" par la Société GENERAL ELECTRIC et "ABIL®" par la Société GOLDSCHMIDT, tel que le produit "Abil 10®" ou bien les produits vendus sous la dénomination de "Q2 1401®" et "Q2 1403®" par la Société DOW CORNING.
Comme polydiorganosiloxanes cycliques selon l'invention, on peut utiliser, seuls ou en mélange, des cyclométhicones de formule:

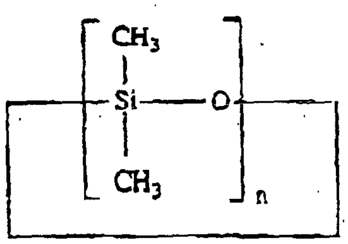

dans laquelle :

n est un nombre entier de 3 à 8.

Parmi les cyclométhicones particulièrement préférées, on citera le cyclotétradiméthylsiloxane (n = 4), le cyclopentadiméthylsiloxane (n = 5), et le cyclohaxadiméthylsiloxane (n = 6).

On peut notamment utiliser les produits vendus sous les dénominations de "DC Fluid 244®", "DC Fluid 245®", "DC Fluid S44®" et "DC Fluid 345®" par la Société DOW CORNING.

D'autres cyclométhicones utilisables selon l'invention sont celles vendues sous les dénominations de "Abil K4®" par la Société GOLDSCHMIDT ; sous les dénominations de "Silbione 70045 V2®" et de "Silbione Huile 70045 V5®" par la Société RHONE POULENC ; ainsi que sous les dénominations de "Volatil Silicone 7158®" et de "Volatil Silicone 7207®" par la Société UNION CARBIDE.

Les organopolysiloxanes selon l'invention peuvent être des alkyl, alcoxy ou phényl-diméthicones tels que, par example:

(a) une alcoxy diméthicone ayant l'une des formules suivantes :

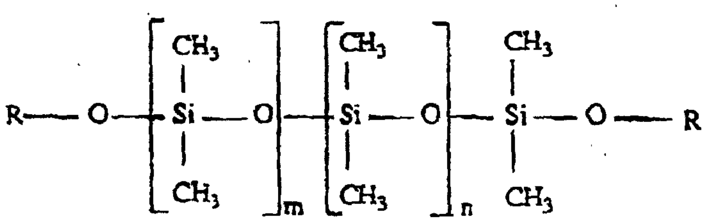

ou

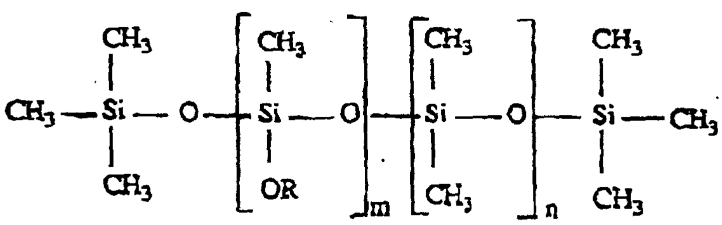

dans lesquelles :

R est un radical alkyle de $C_8$ à $C_{30}$
m est 1 à 100, et n est 0 à 100.

On peut notamment citer le produit vendu sous la dénomination de "Abil wax 2440®" par la Société GOLDSCHMIDT.

(b) une alkyldiméthicone ayant l'une des formules suivantes :

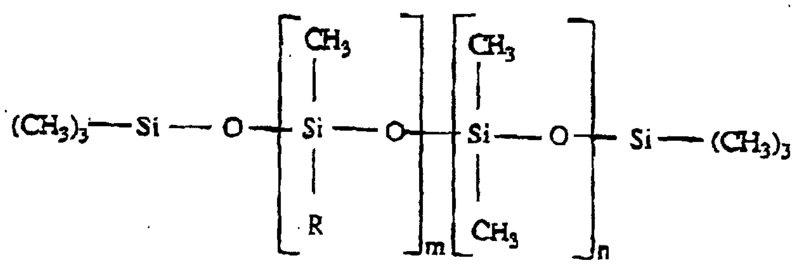

dans laquelle :

R est un radical alkyle de $C_8$ à $C_{30}$,
m est 1 à 100 et n est 0 à 100, ou

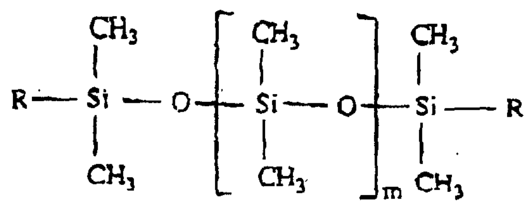

dans laquelle:

R est un raclical alkyle de $C_8$ à $C_{20}$
m est 1 à 100,

(c) une phényl diméthicone ayant la formule suivante:

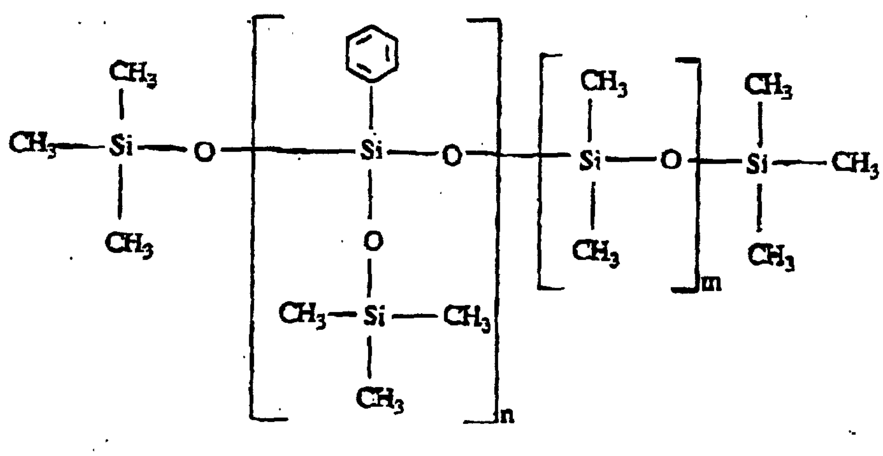

dans laquelle :

m est 0 à 100 et n est 1 à 400
ou la formule suivante:

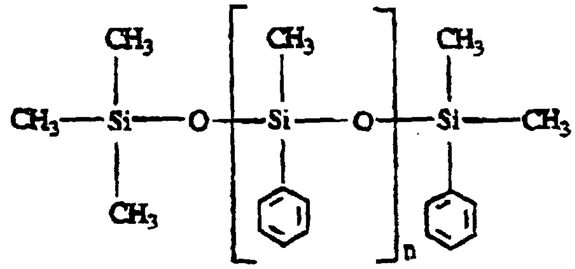

dans laquelle :

n est 0 à 400.

Les organosiloxanes utilisables selon l'invention peuvent également être des triméthylsiloxysilicates (CTFA) renfermant des unités :

$$(R)_2SiO_{2/2}\ ;\ RSiO_{3/2}\ \text{et}\ RSiO_{4/2}$$

R étant un radical alkyle intérieur en $C_1$ à $C_6$ ou phényle.

Tel qu'indiqué ci-dessus, la silicone utilisée selon l'invention est présente en une proportion comprise entre 15 et 40 % mais de préférence comprise entre 15 et 30 % en poids par rapport au poids total de l'émulsion.

Lorsque la silicone est un polydiorganosiloxane éventuellement fonctionnalisé, elle est présente de préférence dans une proportion comprise entre 0,1 et 15 % en poids par rapport au poids total de l'émulsion.

Lorsque la silicone est un polydiorganosiloxane cyclique, elle est présente de préférance en une proportion comprise entre 1 et 30% en poids par rapport au poids total de l'émulsion.

Lorsque la silicone est un organopolysiloxane, elle est présente de préférence en une proportion comprise entre 0,1 et 10 % par rapport au poids total de l'émulsion.

La phase grasse de l'émulsion E/H selon l'invention peut comprendre une ou des huile(s) non siliconée(s) dans une proportion comprise entre 0,1 et 26 %, de préférence entre 0,1 et 15 % en poids par rapport au poids total de l'émulsion.

Comme huile non siliconée, on citera : toute huile (ou mélange d'huiles) fluide stable à la température d'utilisation habituelle des produits cosmétiques et pharmaceutiquement ou cosmétiquement acceptables telles que les huiles végétales ou animales, les huiles minérales ou synthétiques et les triglycérides d'acide gras.

Parmi les huiles végétales ou animales, modifiées ou non, on peut citer par exemple l'huile d'amande douce, l'huile d'avocat l'huile de ricin, l'huile d'olive, l'huile de jojoba, l'huile de tournesol, l'huile de germes de blé, l'huile de sésame, l'huile d'arachide, l'huile de pépins de raisin, l'huile de soja, l'huile de colza, l'huile de carthame, l'huile de coprah, l'huile de maïs, l'huile de noisette, le beurre de karité, l'huile de palme, l'huile de noyau, l'huile de calophylium.

Parmi les huiles minérales, on peut citer par exemple l'huile de vaseline.

Parmi les triglycérides d'acides gras, on peut citer les triglycérides caprylique/caprique, les triglycérides d'acides gras en $C_{10}$ à $C_{18}$ et les triglycérides d'acides gras en $C_{12}$ à $C_{18}$.

L'agent tensio-actif ou émulsionnant tel que défini ci-dessus est utilisé selon l'invention en une proportion comprise entre 0.5 et 10 % et de préférence entre 2 et 6 % en poids par rapport au poids total de l'émulsion et s'est avéré moins irritant que certains autres tensioactifs.

Parmi les produits du commerce pouvant contenir tout ou partie des alkylcicméthicones copolyols utilisables selon l'invention, comme émulsionnant on peut citer notamment ceux vendus sous la dénomination de "Abil WE09®" ou "Abil WS08®" par la Société GOLDSCHMIDT, de "Q2 5200®" par la Société DOW CORNING et de "218 1138®" par la Société GENERAL ELECTRIC.

Selon un mode de réalisation particulier de l'invention, on peut employer un tensioactif additionnel tel qu'un ester ou un éther de glycérol et/ou une dispersion de polydimethylsiloxane oxyéthyléné dans un cyclodiméthylsiloxane ("QS225S®" de la Société DOW CORNING) ayanture HLB comprise entre 2 et 7 et présent en une proportion comprise entre 0.01 et 5 % en poids par rapport au poids total de l'émulsion.

Parmi les tensioactifs additionnels de ce type, on peut citer les esters ou éthers de glycérol notamment l'ester d'acide isostéarique et/ou d'acide succinique et l'éther d'alcool décyltétradécylique. On peut citer par example le produit vendu par la Société DYNAMIT NOBEL sous la dénomination de "Imwitor 780K®" qui est un isostéaryl diglycéryl

succinate.

Il est par ailleurs disponible dans le commerce, certains produits constitués d'un mélange d'alkyldiméthicone copolyol de la formule donnée ci-dessus et d'un tensioactif additionnel du type mentionné ci-dessus.

On citera à cet égard le produit vendu par la Société GOLDSCHMIDT sous la dénomination "Abil WE09®" qui contient un alkyldiméthicone copolyol comportant un rapport en poids des groupes oxyéthylèneloxypropylène compris entre 100:0 et 20:80 associé à un isostéarate de glycérol et du laurate d'hoxyle.

Si désiré et afin de modifier la texture et les propriétés cosméfiques de l'émulsion selon l'invention, la phase grasse peut renfermer au moins un gélifiant huileux choisi parmi:

- les sels métalliques d'acides gras en $C_8$ à $C_{22}$ tels que le stéarate d'aluminium et l'hydroxystéarate d'aluminium et de magnésium.
- les esters d'acides gras en $C_8$ à $C_{22}$ et de glycol,
- les mélanges d'alcools gras en $C_{14}$ à $C_{22}$,
- les cires de silicone de type alkyldiméthicone ou alcoxy diméthicone,
- les argiles organiquement modifiées notamment la bentone,
- les dérivés de cholestérol, notamment l'hydroxycholestérol, et
- les mélanges de ceux-ci.

Les gélifiants huileux peuvent être présents en une proportion très variable selon la texture désirée. Toutefois, dans la plupart des cas, ils sont présents en une proportion comprise entre 0,1 et 10 % en poids par rapport au poids total de l'émulsion.

Selon l'invention, la phase grasse peut également renfermer des pigments éventuellement enrobés par des substances hydrophiles ou hydrophobes tels que:

- le polyéthylène,
- la lécithine,
- un sel d'un acide aminé tel que l'acylglutamate d'aluminium,
- le polyméthylméthacrylate,
- le triisostéaroyltitanate, et
- le collagène,

Parmi les pigments enrobés, on peut citer notamment les pigments vendus sous la dénomination de "Covasil®" par la Société WACKER (pigments au triisostéaroyl-titanate).

Les pigments ainsi enrobée peuvent être incorporés dans l'émulsion selon l'invention en une proportion comprise entre 0,1 et 15 % en poids par rapport au poids total de l'émulsion.

Parmi les autres adjuvants liposolubles que l'on peut incorporer à la phase grasse, on peut citer les filtres U. V. lipophiles, les vitamines lipophiles, les antioxydants et les parfums.

La phase phase aqueuse peut également renfermer des adjuvants communément utilisés dans les émulsions E/H cosmétiques. On citera par exemple les lubrifiants, les agents texturisants du type cire de silicone polyéther, les agents hydratants, tels que le glycérol et la propylène glycol, les protéines ou leurs hydrolysats tels que ceux de collagène et d'élastine, les filtres UV hydrophiles et les polysaccharides ainsi que des électrolytes tels que NaCl ou $MgSO_4$.

L'émulsion selon l'invention peut également incorporer des charges d'origine végétale, minérale ou synthétique, en particulier la poudre d'amidon, de la silices colloïdale, de la poudre de nylon (Orgasol®), et du talc.

Un des principaux avantages découlant de la stabilisation très efficace de l'émulsion selon l'invention par les gélifiants décrits ci-dessus est la possibilité d'incorporer des substanoes réputées destabilisantes des émulsions E/H notamment celles ayant un caractère ionique.

On peut citer notamment les filtres U.V. tels que la benzophénone-4 et certains principes actifs tels que des oligoéléments et des dérivés biologiques. Parmi les oligo-éléments, on citera le gluconate de magnésium et parmi les dérivés biologiques, on citera les hydrolysats de protéine sérique d'origine animale, le pyrrolidone carboxylate de sodium.

Les émulsions selon l'invention peuvent être préparées par tout procédé classique.

Toutefois, il est préférable d'obtenir l'émulsion de la manière suivante: on chauffe la phase grasse contenant l'émulsionnant jusqu'à une température suffisante pour fondre tous les constituants, de préférence entre 20 et 95°C, et on incorpore les adjuvants liposolubles désirés.

Ensuite, on ajoute à la phase grasse fondue, sous forte agitation, par example à l'aide d'un agitateur de type MORITZ la phase aqueuse contenant le gélifiant portée à une température comprise entre 20 et 95°C dans laquelle tous les adjuvants hydrosolubles désirés ont préalablement été incorporés.

Les émulsions selon l'invention peuvent être sous forme de crème blanche ou teintée, sous forme de fond de teint, de mascara, de blush ou d'un produit de maquillage pour les lèvres. Elles se conservent bien même en l'absence d'agent de conservation.

L'invention sera maintenant illustrée par les examples suivants sans pour autant la limiter.

**EXEMPLE 1** : *Crème blanche*

| Phase grasse A : | |
|---|---|
| - Alkyl diméthicone copolyol "WE08®" de la Société GOLDSCHMIDT | 5 % |
| - "Mygliol 812®" de la Société DYNAMIT NOBEL | 4 % |
| - Ester de glycérol "imwitor 780K®" de la Société DYNAMIT NOBEL | 2 % |
| - Silicone "Q2 1401®" de la Société DOW CORNING | 0,5 % |
| - Silicone volatile "Fluid 245®" de la Société DOW CORNING | 18,5 % |

| Phase aqueuse B : | |
|---|---|
| - Polyacrylate d'ammonium | 1 % |
| - Glycérine | 15 % |
| - $MgSO_4$ | 0,7 % |
| - Conservateur | QS |
| - Eau | 53,3 % |

On obtient la crème blanche de la manière suivante : on chauffe la phase B à une température d'environ 90°C et on l'ajoute en plusieurs fractions à la phase A portée à la même température. Le mélange est effectué à l'aide d'une turbine de type Moritz à une vitesse d'environ 3000 t/mn.

La crème obtenue est d'un bel aspect, brillante, légèrement translucids, de toucher agréable, doux, non collant et frais.

**EXEMPLE 2** : *Fond de teint*

| Phase A : | |
|---|---|
| - Alkyl diméthicone copolyol "WE09®" de la Société GOLDSCHMIDT | 5 % |
| - Alkyldiméthicorte "Abil Wax 9810®" de la Société GOLDSCHMIDT | 2 % |
| - Silicone "Abil 10®" de la Société GOLDSCHMIDT | 9 % |
| - Oxydes de titane enrobés par des lipoaminoacides de la Société MAPRECOS | 4,12 % |
| - Oxydes de fer enrobés par des lipoaminoacides de la Société MAPRECOS | 0,88 % |
| - Silicone volatile "Fluid 245®" de la Société DOW CORNING | 13,5 % |

| Phase B : | |
|---|---|
| - Carboxyméthylcellulose | 0,5 % |
| - Glycérine | 14 % |
| - Polyéthylène glycol-20 de poids moléculaire 1000 "Carbonax 1000®" de la Société UNION CARBIDE | 1,7 % |
| - $MgSO_4$ | 0,7 % |
| - Conservateur | 0,5 % |
| - Eau | 48,1 % |

On obtient le fond de teint de la manière suivante : on homogénéise l'ensemble de la phase A par une turbine de type Moritz à 90°C à une vitesse d'environ 3000 t/mn.

Ensuite, on chauffe la phase B à 90°C et on l'introduit en plusieurs fractions dans la phase A par agitation vigoureuse.

Le fond de teint obtenu après refroidissement présente un très bel aspect et de bonnes qualités cosmétiques.

**EXEMPLE 3** : *Crème blanche*

| Phase A : | |
|---|---|
| - Alkyldiméthicone copolyol "WE 09®" de la Société GOLDSCHMIDT | 2 % |
| - Silicone volatile "Fluid 245®" de la Société DOW CORNING | 17 % |
| - "Mygliol 812®" de la Société DYNAMIT NOBEL | 4 % |
| - Ester de glycérol "Imwitor 780 K®" de la Société DYNAMIT NOBEL | 1 % |
| - Silicone "Q2 1403®" de la Société DOW CORNING | 4 % |

| Phase B: | |
|---|---|
| - Glycérine | 15 % |
| - Copolymère d'acrylate d'ammonium | 1 % |
| - $MgSO_4$ | 0,7 % |
| - Consevateur | QS |
| - Eau | 55,3 % |

On obtient cette crème de la même manière que pour l'exemple 1.

**EXEMPLE 4** : *Crème blanche*

| Phase A : | |
|---|---|
| - Alkyldiméthicone copolyol "WE 09®" de la Société GOLDSCHMIDT | 5 % |
| - Silicone volatile "Fluid 245®" de la Société DOW CORNING | 12 % |
| - Silicone "Q2 1401®" de la Société DOW CORNING | 4 % |
| - "Mygliol 812®" de la Société DYNAMIT NOBEL | 2 % |
| - Ester de glycérol "imwitor 780 K®" de la Société DYNAMIT NOBEL | 2 % |
| - Sel mixte d'aluminium et de magnésium d'acides gras "Gilugel Sil®" de la Société GIULINI CHEMIE | 3 % |

| Phase B: | |
|---|---|
| - Glycérine | 15 % |
| - Copolymère d'acrylate d'ammonium "PAS 5161®" de la Société HOECHST | 0,5 % |
| - $MgSO_4$ | 0,7 % |
| - Conservateur | QS |
| - Eau | 55,8 % |

On obtient cette crème de la même manière que pour l'exemple 1.

**EXEMPLE 5** : *Crème planche*

| Phase A : | |
|---|---|
| - Alkyl diméthicone copolyol "WE09®" de la Société GOLDSCHMIDT | 5 % |
| - Silicone volatile "Fluid 245®" de la Société DOW CORNING | 15 % |
| - "Mygilol 812®" de la Société DYNAMIT NOBEL | 4 % |
| - Silcone "O2 1401®" de la Société DOW CORNING | 4 % |
| - Ester de glycérol "Imwitor 780 K®" | 2 % |
| - Conservateur | QS |

| Phase B : | |
|---|---|
| - $MgSO_4$ | 0,70 % |
| - Kératine sulfonique | 2 % |
| - Eau | 67,3 % |

On obtient cette crème de la même manière qu'à l'exemple 1.

**EXEMPLE 6** : *Fond de teint fluide*

| Phase A : | |
|---|---|
| - Alkyl diméthicone copolyol "WE 09®" de la Société GOLDSCHMIDT | 10 % |
| - Cyclopentadiméthylsiloxane "D5®" de la Société DOW CORNING | 30 % |
| - Oxyde de titane enrobé de silicones de la Société WAGKER | 4,8 % |
| - Oxyde de fer noir enrobé de silicones de la Société WACKER | 0,22 % |
| - Oxyde de fer rouge enrobé de silicones de la Société WACKER | 0,25 % |
| - Oxyde de fer jaune enrobé de silicones de la Société WACKER | 1,43 % |
| - Poudre de nylon "Orgasol®" de la Société ATOCHEM | 5 % |

| Phase B: | |
|---|---|
| - $MgSO_4$ | 1 % |
| - Carboxyméthylcellulose | 0,5 % |
| - Cire de silicone polyéther "SLM 23008®" de la Société WACKER | 3 % |
| - Polyéthylèneglycol-20 (Carbowax 1000®) | 2 % |
| - Polyéthylèneglycol-8 (Carbowax 400®) | 3 % |
| - Conservateur | 0,3 % |
| - Eau qsp | 100 % |

Le fond de teint est fluide, il s'étale facilement et laisse la peau très uniforme, mate, naturelle et douce.

**EXEMPLE 10 :** *Fond de teint E/H siliconée souple*

| Phase A: | |
|---|---|
| - Alkyl diméthicone copolyol "WE 09®" de la Société GOLDSCHMIDT | 5 % |
| - Cyclopentadiméthylsilocane "D5®" de la Société DOW CORNING | 28 % |

(suite)

| Phase A: | |
|---|---|
| - Polyphénylsiloxane "RP761®" de la Sociétée RHONE POULENC | 0,3 % |
| - Hydroxystéarate d'Al et de Mg en suspension dans du cyclométhicone "Multigel SIL5®" de la Société GIULINI CHEMIE | 6 % |
| - Oxyde de titane enrobé de silicones de la Société WACKER | 4,8 % |
| - Oxyde de fer noir enrobé desilicones de la Société WACKER | 0,22 % |
| - Oxyde de fer rouge enrobé de silcones de la Société WACKER | 0,55 % |
| - Oxyde de fer jaune enrobé de silicones de la Société WACKER | 1,43 % |
| - Microbilles de résine siliconée "Tospearl 108®" | 6% |

| Phase B : | |
|---|---|
| - $MgSO_4$ | 1 % |
| - Carboxyméthylcellulose | 0,3 % |
| - Polyéthylèneglycol- 8(Carbowax 400®) | 2 % |
| - Conservateur | 0,8 % |
| - Eau qsp | 100 % |

Le fond de teint se présente sous forme d'une crème souple très fine. Après application, la peau est douce et mate.

## Revendications

1. Emulsion eau-dans-huile stable à usage cosmétique ou pharmaceutique **caractérisée par le fait qu'**elle comprend une phase grasse en une proportion de 15 à 40 % constituée d'au moins une silicone à raison de 15 à 40 % en poids par rapport au poids total de l'émulsion et une phase aqueuse contenant au moins un gélifiant aqueux organique insensible aux électrolytes choisi dans le groupe constitué par des formes naturelles, le proteins, les hydrolizats de proteins, les dérivés acryliques, les polyéthylènephycol et leurs melanges l'agent émulationnant de ladite émulsion étant un alkyl- ou alcoxy-diméthicone copolyol de formule générale :

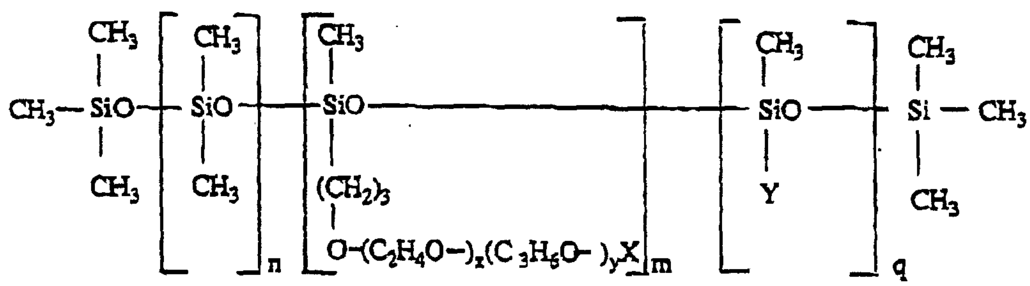

dans laquelle :

X est un atome d'hydrogène, un alkyle, un alcoxy au un acyle, en $C_1$-$C_{16}$,
Y est un radical alkyle ou alcoxy en $C_8$ à $C_{22}$,
n = 0 à 200,
m = 1 à 40,
q = 1 à 100,
le poids moléculaire du reste $(C_2H_4O-)_x(C_3H_6O-)_y$-X étant de 250 à 2000, x et y étant choisis de telle sorte que le rapport en poids des groupes oxyéthylène/oxypropylène soit compris entre 100:0 et 20:80.

**2.** Emulsion selon la revendication 1, **caractérisée par le fait que** la viscosité de l'émulsion est comprise entre 0,1 Pa.sec et 10 Pa.sec et de préférence entre 0,2 Pa.sec et 6 Pa.sec.

**3.** Emulsion selon la revendication 1, **caractérisée par le fait que** la silicone est un polydiorganosiloxane linéaire, éventuellement fonctionnalisé, ou cyclique, ou un organopolysiloxane ou un mélange de ceux-ci.

**4.** Emulsion selon la revendication 3, **caractérisée par le fait que** le polydiorganosiloxane linéaire répond à la formule :

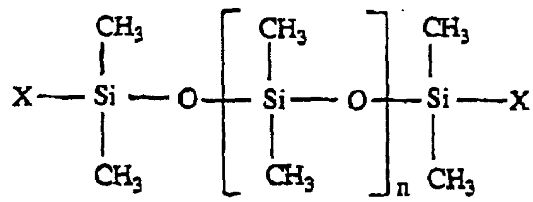

dans laquelle :

X est $-CH_3$ ou OH et n = 0 à 5000
et que le polydiorganosiloxane cyclique répond à la formule :

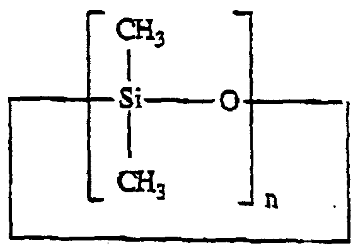

**5.** Emulsion selon la revendication 3, **caractérisée par le fait que** l'organopolysiloxane est choisi dans le groupe constitué par les alkyl, alcoxy et phényl-diméthicones.

**6.** Emulsion selon l'une quelconque des revendications précédentes, **caractérisée par le fait que** la phase grasse est en outre constituée d'une huile non silliconnée à raison de 0,1 à 25% en poids par rapport au poids total de l'émulsion.

**7.** Emulsion selon l'une quelconque des revendications précédentes, **caractérisée par le fait que** l'alkyl- ou alcoxy-diméthicone copolyol est présent en une proportion comprise entre 0,5 et 10% et de préférence entre 2 et 8 % en poids par rapport au poids total de l'émulsion.

**8.** Emultion selon l'une quelconque des revendications précedentes **caractérisée par le fait que** le gélifiant aqueux est choisi parmi les gommes de xanthane, de guar, de caroube, les scléroglucans, les dérivés de chitine ou de chitosane, le polyacrylate de glycérol, le copolymère d'acylate d'ammonium les polyéthyléniglycole et leurs mélanges.

**9.** Emulsion selon l'une quelconque des revendications précédentes, **caractérisée par le fait que** le gélifiant aqueux est présent en une proportion comprise entre 0,1 et 5 % en poids par rapport au poids total de l'émulsion.

**10.** Emulsion selon l'une quelconque des revendications précédentes, **caractérisée par le fait que** la phase grasse contient un gélifiant huileux choisi parmi les sels métalliques d'acides gras en $C_8$ à $C_{22}$, les esters d'acides gras et de glycol, les mélanges d'alcools gras en $C_{14}$ à $C_{32}$, les cires de silicone, les argiles organiquement modifiées, les dérivés de cholestérol et leurs mélanges.

**11.** Emulsion selon l'une quelconque des revendications précédentes, **caractérisée par le fait que** le gélifiant huileux

est présent en une proportion comprise entre 0,1 et 10 % en poids par rapport au poids total de l'émulsion.

**12.** Emulsion selon l'une quelconque des revendications précedentes, **caractérisée par le fait qu'**elle comprend comme tensioactif supplémentaire un ester ou éther de glycérol et/ou une dispersion de polydiméthylsiloxane oxyéthyléné dans un cyclodiméthylsiloxane ayant une HLB comprise entre 2 et 7 présent en une proportion comprise entre 0,01 et 5 % en poids par rapport au poids total de l'émulsion.

**13.** Emulsion selon l'une quelconque des revendications précédentes, **caractérisée par le fait que** la phase grasse comprend en outre au moins un adjuvant liposoluble choisi parmi les filtres U.V. lipophiles, les vitamines lipophiles, les antioxydants et les parfums.

**14.** Emulsion selon l'une quelconque des revendications précédentes, **caractérisée par le fait que** la phase aqueuse comprend en outre au moins une substance hydrosoluble choisie parmi les hydratants, les lubrifiants, les texturisants, les protéines ou leurs hydrolysats, les polysaccharidès, les électrolytes, les filtres U.V. hydrophiles, les oligoéléments et les dérivés biologiques.

**15.** Emulsion selon l'une quelconque des revendications précédentes, **caractérisée par le fait qu'**elle est exemple de conservateur.

**16.** Emulsion selon l'une quelconque des revendications précédentes, **caractérisée par le fait qu'**elle renferme des pigments éventuellement enrobés par des substances hydrophiles ou hydrophobes.

**17.** Emulsion selon la revendication 16, **caractérisée par le fait que** les substances hydrophiles ou hydrophobes d'enrobage sont choisies parmi le polyéthylène, la lécithine, les sels d'acides aminés, le polyméthylméthacrylate, le triisostéaroyltitanate et le collagène.

## Claims

**1.** Stable water-in-oil emulsion, for cosmetic or pharmaceutical use, **characterized in that** it comprises a fatty phase in a proportion of 15 to 40% consisting of at least one silicone in a proportion of 15 to 40% by weight relative to the total weight of the emulsion, and an aqueous phase containing at least one organic electrolyte-insensitive aqueous gelling agent chosen from the group consisting of natural gums, proteins, protein hydrolysates, acrylic derivatives, polyethylene glycols and mixtures thereof, the emulsifying agent of the said emulsion being an alkyl- or alkoxydimethicone copolyol of general formula:

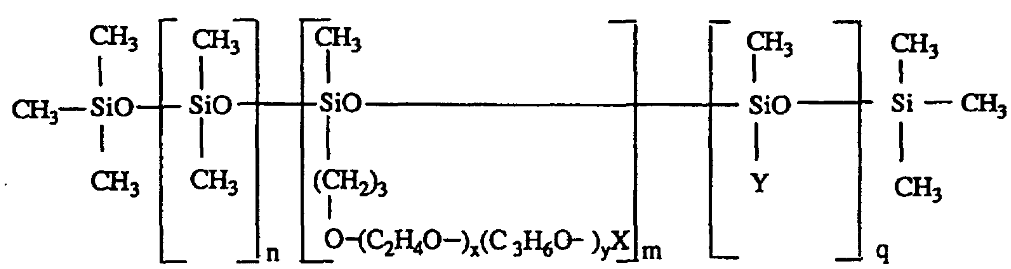

in which:

X is a hydrogen atom or a $C_1$-$C_{16}$ alkyl, alkoxy or acyl,
Y is a $C_8$ to $C_{22}$ alkyl or alkoxy radical,
n = 0 to 200,
m = 1 to 40,
q = 1 to 100,
the molecular weight of the residue $(C_2H_4O-)_x(C_3H_6O-)_y$-X being from 250 to 2000, and x and y being chosen in such a way that the weight ratio of oxyethylene to oxypropylene groups is between 100:0 and 20:80.

**2.** Emulsion according to Claim 1, **characterized in that** the viscosity of the emulsion is between 0.1 Pa.sec and 10 Pa.sec, and preferably between 0.2 Pa.sec and 6 Pa.sec.

**3.** Emulsion according to Claim 1, **characterized in that** the silicone is an optionally functionalized linear polydiorganosiloxane or a cyclic polydiorganosiloxane, an organopolysiloxane or a mixture of these.

**4.** Emulsion according to Claim 3, **characterized in that** the linear polydiorganosiloxane corresponds to the formula:

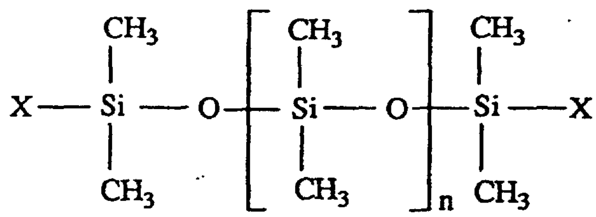

in which:

X is $-CH_3$ or OH and n = 0 to 5000,
and **in that** the cyclic polydiorganosiloxane corresponds to the formula:

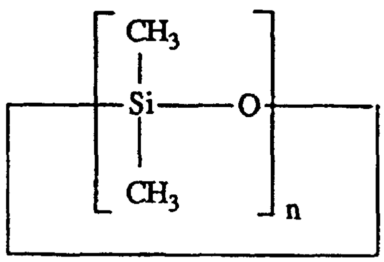

in which:
n is an integer from 3 to 8.

**5.** Emulsion according to Claim 3, **characterized in that** the organopolysiloxane is chosen from the group consisting of alkyl-, alkoxy- and phenyldimethicones.

**6.** Emulsion according to any one of the preceding claims, **characterized in that** the fatty phase consists, in addition, of a non-silicone oil in a proportion of 0.1 to 25 % by weight relative to the total weight of the emulsion.

**7.** Emulsion according to any one of the preceding claims, **characterized in that** the alkyl- or alkoxydimethicone copolyol is present in a proportion of between 0.5 and 10%, and preferably between 2 and 6%, by weight relative to the total weight of the emulsion.

**8.** Emulsion according to any one of the preceding claims, **characterized in that** the aqueous gelling agent is chosen from xanthan, guar and carob gums, scleroglucans, chitin or chitosan derivatives, glycerol polyacrylate, ammonium acrylate copolymer, polyethylene glycols and mixtures thereof.

**9.** Emulsion according to any one of the preceding claims, **characterized in that** the aqueous gelling agent is present in a proportion of between 0.1 and 5% by weight relative to the total weight of the emulsion.

**10.** Emulsion according to any one of the preceding claims, **characterized in that** the fatty phase contains an oily gelling agent chosen from metal salts of $C_8$ to $C_{22}$ fatty acids, esters of fatty acids and glycol, mixtures of $C_{14}$ to $C_{32}$ fatty alcohols, silicone waxes, organically modified clays, cholesterol derivatives and mixtures thereof.

**11.** Emulsion according to any one of the preceding claims, **characterized in that** the oily gelling agent is present in a proportion of between 0.1 and 10% by weight relative to the total weight of the emulsion.

**12.** Emulsion according to any one of the preceding claims, **characterized in that** it comprises as additional surfactant a glycerol ester or ether and/or a dispersion of oxyethylenated polydimethylsiloxane in a cyclodimethylsiloxane having an HLB of between 2 and 7, present in a proportion of between 0.01 and 5% by weight relative to the total weight of the emulsion.

**13.** Emulsion according to any one of the preceding claims, **characterized in that** the fatty phase comprises, in addition, at least one fat-soluble adjuvant chosen from lipophilic UV screening agents, lipophilic vitamins, antioxidants and perfumes.

**14.** Emulsion according to any one of the preceding claims, **characterized in that** the aqueous phase comprises, in addition, at least one water-soluble substance chosen from hydrating agents, lubricants, texturing agents, proteins or their hydrolysates, polysaccharides, electrolytes, hydrophilic UV screening agents, trace elements and biological derivatives.

**15.** Emulsion according to any one of the preceding claims, **characterized in that** it is free from preservative.

**16.** Emulsion according to any one of the preceding claims, **characterized in that** it contains pigments, where appropriate coated with hydrophilic or hydrophobic substances.

**17.** Emulsion according to Claim 16, **characterized in that** the hydrophilic or hydrophobic coating substances are chosen from polyethylene, lecithin, amino acid salts, poly(methyl methacrylate), triisostearoyl titanate and collagen.

## Patentansprüche

**1.** Stabile Wasser-in-Öl-Emulsion für den kosmetischen oder pharmazeutischen Gebrauch, **dadurch gekennzeichnet, dass** sie eine Fettphase in einem Mengenverhältnis von 15 zu 40 Gew.-%, die aus mindestens einem Silikon in einem Mengenverhältnis von 15 bis 40 Gew.-% besteht, bezogen auf das Gesamtgewicht der Emulsion, sowie eine wässrige Phase umfasst, enthaltend mindestens ein wässriges organisches Geliermittel, unempfindlich gegenüber Elektrolyten, ausgewählt aus der Gruppe, bestehend aus natürlichen Gummen, Proteinen, Proteinhydrolysaten, Acrylsäurederivaten, Polyethylenglykolen und deren Mischungen, wobei der Emulgator in der Emulsion ein Alkyl- oder Alkoxydimethicon-Copolyol gemäß der folgenden allgemeinen Formel darstellt:

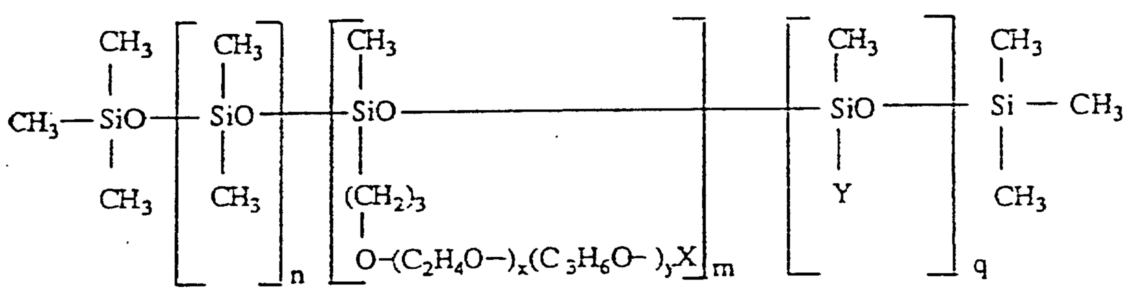

worin:

X ein Wasserstoffatom, einen Alkyl-, Alkoxy- oder Acylrest mit jeweils $C_1$-$C_{16}$ bedeutet,
Y einen Alkyl- oder Alkoxyrest mit jeweils $C_8$-$C_{22}$ darstellt,
n = 0 bis 200
m = 1 bis 40
q = 1 bis 100
bedeuten, und wobei das Molekulargewicht des Restes

$$(C_2H_4O-)_X(C_3H_6O-)_Y-X$$

250 bis 2000 beträgt, und x und y in der Weise ausgewählt sind, dass das Gewichtsverhältnis der Oxyethylen/Oxypropylen-Gruppen zwischen 100 : 0 und 20 : 80 beträgt

**2.** Emulsion nach Anspruch 1, **dadurch gekennzeichnet, dass** die Viskosität der Emulsion zwischen 0,1 Pa sec und 10 Pa sec, vorzugsweise zwischen 0,2 Pa · sec und 6 Pa • sec beträgt.

**3.** Emulsion nach Anspruch 1, **dadurch gekennzeichnet, dass** das Silikon ein gegebenenfalls funktionalisiertes lineares Polydiorganosiloxan oder cyclisches Polydiorganosiloxan bzw. ein Organopolysiloxan oder eine Mischung daraus darstellt.

**4.** Emulsion nach Anspruch 3, **dadurch gekennzeichnet, dass** das lineare Polydiorganosiloxan der folgenden Formel entspricht:

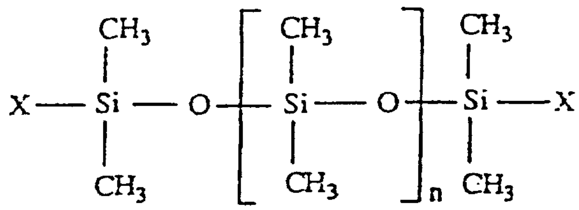

worin:

X $-CH_3$ oder OH und n = 0 bis 5000 bedeuten, und das cyclische Polydiorganosiloxan der folgenden Formel entspricht:

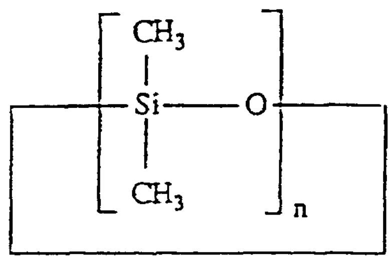

worin:
n eine ganze Zahl zwischen 3 bis 8 bedeutet.

**5.** Emulsion nach Anspruch 3, **dadurch gekennzeichnet, dass** das Organopolysiloxan aus der Alkyl-, Alkoxy- und Phenyldimethiconen bestehenden Gruppe ausgewählt ist.

**6.** Emulsion nach einem der vorstehenden Ansprüche, **dadurch gekennzeichnet, dass** die Fettphase zusätzlich noch aus einem von Silikon verschiedenen Öl in einem Mengenverhältnis von 0,1 bis 25 Gew.-% in Bezug auf das Gesamtgewicht der Emulsion besteht.

**7.** Emulsion nach einem der vorstehenden Ansprüche, **dadurch gekennzeichnet, dass** das Alkyl- oder Alkoxydimethicon-Copolyol in einem Verhältnis zwischen 0,5 und 10 Gew.-%, vorzugsweise zwischen 2 und 8 Gew.-% in Bezug auf das Gesamtgewicht der Emulsion vorhanden ist.

**8.** Emulsion nach einem der vorstehenden Ansprüche, **dadurch gekennzeichnet, dass** das wässrige Geliermittel ausgewählt ist unter Xanthan-, Guar-, Johannisbrot-, Scleroglucan-, Chitinderivat- und Chitosangummen, den Polyacrylaten von Glycerin, Ammoniumacrylatcopolymeren, Polyethylenglycolen und deren Mischungen.

**9.** Emulsion nach einem der vorstehenden Ansprüche, **dadurch gekennzeichnet, dass** das wässrige Geliermittel in einem Verhältnis zwischen 0,1 und 5 Gew.-% in Bezug auf das Gesamtgewicht der Emulsion vorhanden ist.

**10.** Emulsion nach einem der vorstehenden Ansprüche, **dadurch gekennzeichnet, dass** die Fettphase ein öliges Geliermittel enthält, das aus $C_8$-$C_{22}$-Fettsäuremetallsalzen, Fettsäureglykolestern, Gemischen aus $C_{14}$-$C_{32}$-Fettalkoholen, Silikonwachsen, organisch modifizierten Tonen, Cholesterinderivaten sowie deren Gemischen ausgewählt ist.

**11.** Emulsion nach einem der vorstehenden Ansprüche, **dadurch gekennzeichnet, dass** das ölige Geliermittel in einem Verhältnis zwischen 0,1 und 10 Gew.-% in Bezug auf das Gesamtgewicht der Emulsion vorhanden ist.

**12.** Emulsion nach einem der vorstehenden Ansprüche, **dadurch gekennzeichnet, dass** sie als zusätzliches Tensid einen Glycerinester oder -ether und/oder eine Dispersion aus oxyethyliertem Polydimethylsiloxan in einem Cyclodimethylsiloxan mit einem HLB-Wert zwischen 2 und 7 ausweist, die jeweils in einem Verhältnis zwischen 0,01 und 5 Gew.-% in Bezug auf das Gesamtgewicht der Emulsion vorhanden sind.

**13.** Emulsion nach einem der vorstehenden Ansprüche, **dadurch gekennzeichnet, dass** die Fettphase zusätzlich noch mindestens einen fettlöslichen Hilfsstoff aufweist, der unter lipophilen UV-Filtersubstanzen, lipophilen Vitaminen, Antioxidantien und Duftstoffen ausgewählt ist.

**14.** Emulsion nach einem der vorstehenden Ansprüche, **dadurch gekennzeichnet, dass** die wässrige Phase zusätzlich noch mindestens eine wasserlösliche Substanz aufweist, die unter Hydratisierungsmitteln, Gleitmitteln, texturbildenden Mitteln, Proteinen oder deren Hydrolysaten, Polysacchariden, Elektrolyten, hydrophilen UV-Filtersubstanzen, Spurenelementen und biologischen Derivaten ausgewählt ist.

**15.** Emulsion nach einem der vorstehenden Ansprüche, **dadurch gekennzeichnet, dass** sie frei von Konservierungsmitteln ist.

**16.** Emulsion nach einem der vorstehenden Ansprüche, **dadurch gekennzeichnet, dass** sie gegebenenfalls mit hydrophilen oder hydrophoben Substanzen überzogene Pigmente miteinschließt.

**17.** Emulsion nach Anspruch 16, **dadurch gekennzeichnet, dass** die hydrophilen oder hydrophoben Umhüllungssubstanzen unter Polyethylen, Lecithin, Aminosäuresalzen, Polymethylmethacrylat, Triisostearyltitanat und Collagen ausgewählt sind.